# EUROPEAN PATENT APPLICATION

(11) **EP 1 944 299 A1**
(43) Date of publication of application: **16.07.2008**
(21) Application number: 07250111.7
(22) Date of filing: 11.01.2007
(51) Int. Cl.: C07D 301/10, B01J 23/50, B01J 23/847, B01J 35/00

(54) **Catalyst system and process for the production of epoxides**

(71) Applicant: INEOS EUROPE LIMITED, Lyndhurst, Hampshire, SO43 7FG (GB)
(72) Inventor: The designation of the inventor has not yet been filed
(74) Representative: King, Alex

(57) **Abstract**

The present invention relates to a catalyst system which is a mixture of at least two catalytic species, the first catalytic species providing dehydrogenation activity and the second catalytic species providing epoxidation activity and comprising silver. The present invention also relates to a process for the production of epoxides, in particular a process for the production of an epoxide from a mixture comprising an alkane and an alkene, which process comprises contacting said mixture comprising said alkane and said alkene and a source of oxygen with such a catalyst system comprising a mixture of at least two catalytic species, the first catalytic species providing dehydrogenation activity and the second catalytic species providing epoxidation activity and comprising silver.

## Description

The present invention relates to a catalyst system which is a mixture of at least two catalytic species, and also to a process for the production of epoxides, in particular for the production of epoxides from a mixture of an alkane and an alkene using said catalyst system.

The production of epoxides from alkenes by partial oxidation over a suitable catalyst is a well-known and widely operated commercial process.

The epoxidation of ethylene to ethylene oxide for example is believed to operate commercially exclusively using supported silver epoxidation catalysts.

An alternative desired route to epoxides would be from the corresponding alkanes. There are significant incentives to develop such a process due to the relatively low cost of alkanes compared to alkenes, but the low reactivity of alkanes to epoxidation has limited the developments in this area.

It has now been found that an epoxide can be formed from an alkane by contacting a mixture of an alkane and an alkene with a mixed catalytic bed in a reactor.

Thus, in a first aspect, the present invention provides a process for the production of an epoxide from a mixture comprising an alkane and an alkene, which process comprises contacting said mixture comprising said alkane and said alkene and a source of oxygen with a catalyst system comprising a mixture of at least two catalytic species, the first catalytic species providing dehydrogenation activity and the second catalytic species providing epoxidation activity and comprising silver.

The present invention provides an improved process for the production of an epoxide which uses a catalyst system which is a mixture of at least two catalytic species to produce an epoxide from the corresponding alkane.

"Mixture of at least two catalytic species", as used herein, means that the catalytic species providing dehydrogenation activity and the catalytic species providing epoxidation activity are mixed in the catalyst system, rather than provided sequentially. In effect, the mixture combines a "conventional" epoxidation catalyst with a dehydrogenation catalyst, and reference herein to "catalysts" should therefore be understood to also equate to "catalytic species". In particular, the first catalytic species will hereinafter be referred to as a dehydrogenation catalyst and the second catalytic species will hereinafter be referred to as an epoxidation catalyst.

In one embodiment, the "mixture of at least two catalytic species" may be achieved by having an intimate mixture of the two separate catalysts, for example of the two catalysts on separate particles of suitable supports. In an alternative embodiment both the dehydrogenation and epoxidation catalysts (catalytic species) may be supported at different locations on a single support e.g. on the same particles, or on a larger substrate, such as a monolith or a foam to provide the mixed catalyst bed.

The catalyst system is preferably provided in the form of a fixed catalyst bed. The catalyst bed may be provided as a uniformly mixed catalyst bed or may be graded, for example such that more of one catalyst compared to the other is present at the front of the mixed catalyst bed than at the rear of the mixed catalyst bed. Alternatively, or additionally, further catalyst beds comprising increased relative levels of dehydrogenation or epoxidation functionality compared to the mixed catalyst bed may be present before or after the mixed catalyst bed

The process of the present invention allows use of a lower cost feed than a "pure" alkene, such as ethylene, but requires only a single reactor for both dehydrogenation and epoxidation reactions (avoiding any intermediate product treatment or separation that might be required in a two-stage process).

In particular, it is a significant advantage of the present invention that a mixed alkane and alkene containing feed can be used, and that the overall reaction is conversion of an alkane to an epoxide rather than an alkene. Since alkane feeds are generally much cheaper than alkenes it has long been desired to convert alkanes directly to epoxides, but using mixed feeds according to the present invention can be more advantageous still. In particular, "pure" alkene or alkane feeds are often formed in the first place by treatment of mixed alkane/alkene streams e.g. cracker product streams, to separate alkanes and alkenes of the same number of carbon atoms from each other e.g. ethane from ethylene. This separation is costly. By using a mixed stream according to the present invention such a separation step may not be required at all, or, even if some separation is still required, the separation step may be operated at much lower severity.

The alkene in the mixture comprising an alkane and an alkene may also be provided by recycle of unreacted alkene from the process of the present invention. In fact, the reaction of the present invention typically produces a mixture of epoxide, alkene and unreacted alkane, which alkene and alkane can be recycled after separation of the epoxide, and mixed with a "fresh" alkane containing feed regardless of whether the "fresh" feed also comprises alkene in the first place.

For avoidance of doubt, both alkane and alkene in the mixture comprising an alkane and an alkene will have the same number of carbon atoms. Thus, the mixture comprising an alkane and an alkene may comprise a mixture of butane and butylene, a mixture of propane and propylene or a mixture of ethane and ethylene. Mixtures comprising propane and propylene or comprising ethane and ethylene are preferred, with a mixture comprising ethane and ethylene being most preferred.

The source of oxygen may be any suitable source of oxygen which provides oxygen for reaction. Examples of suitable sources are molecular oxygen-containing gases, such as molecular oxygen itself and air, peroxides, such as hydrogen peroxide, ozone and nitrogen oxides, such as nitrogen monoxide (N₂O).

Other feed components, especially those conventionally fed to an epoxidation reaction, such as inert carrier gases and halogenated hydrocarbons may also be fed to the process if desired.

The process may be performed at any suitable pressure, but is preferably performed at an elevated pressure, especially of from 5 to 40 barg, more preferably of from 10 to 25 barg.

The process is usually performed at a temperature of at least 100°C. The temperature is usually less than 500 °C. Temperatures in the range 200 °C to 350 °C are preferred.

The epoxidation catalyst comprises silver but may otherwise be any suitable epoxidation catalyst. The epoxidation catalyst may be supported on any suitable support, such as ceria, silica, silicon carbide, zirconia, zeolites, titania or alumina, but is preferably supported on an alumina, most preferably on an alpha alumina. The silver may be promoted, for example with rhenium and/or other promoters suitable for the epoxidation reaction.

Any suitable dehydrogenation catalyst may be used. Preferred dehydrogenation catalysts are metal oxides (MO's). Preferably the metal oxide catalyst comprises one or more of Cr, V, Nb, Pd, Pt, Ni, Co, Rh, Ir, Fe, Ru, Cu, Zn and Au. The most preferred dehydrogenation catalyst comprises a mixture of nickel and niobium.

The dehydrogenation catalyst can be a supported or an unsupported catalyst.

Where the dehydrogenation catalyst is a supported catalyst, it is preferably a supported metal oxide catalyst. Where the catalyst is supported and on a physically different support material to the epoxidation catalyst, the support used may be any suitable support, independent of that used for the epoxidation catalyst. Suitable supports include ceria, silica, silicon carbide, zirconia, zeolites, niobia, mesoporous materials such as MCM, SBA (mesoporous silicas) and MOF (metal-organic framework) materials, silicalites, titania or alumina. Silicas and aluminas are most preferred.

For avoidance of doubt, as used herein, the term "dehydrogenation" includes oxidative dehydrogenation. Particularly preferred oxidative dehydrogenation catalysts are unsupported multi-component bulk metal oxides, such as described in WO 00/48971.

The catalysts may be produced by any suitable technique or techniques. One suitable technique for producing supported catalysts is wet impregnation, typically followed by calcination. Additives, such as phosphorous containing salts, may be added to increase catalyst dispersion during impregnation. The catalysts may also be produced by grafting of catalytic species to a support, for example as described in C. Copéret et al., Angew. Chem. Int. Ed., 2003, 42(2), 156, followed by calcination. This results directly in highly dispersed catalyst species.

As a further advantage of the process of the present invention, the ability to produce an epoxide from a mixture comprising an alkane and an alkene also allows the present process to be advantageously integrated with a number of different alkane sources.

As one example, where the present invention uses ethane which is formed as a byproduct in the steam-cracking of naphtha, this ethane is conventionally recycled into the cracker. As well as the possibility to remove, or at least reduce, the requirement for an ethane/ethylene separation on such a stream, use of this ethane in the process of the present invention would have the additional beneficial effect of releasing furnace capacity for fresh naphtha cracking.

As a further example, the present invention may use ethane derived from natural gas. This ethane could be used with a significant quantity of methane present, with the methane acting as a carrier gas/diluent, as is conventionally used for current ethylene oxide technology. This therefore has the advantage that a feed may be used which does not require complete separation of methane.

In a second aspect, the present invention also provides a catalyst system which is a mixture of at least two catalytic species the first catalytic species providing dehydrogenation activity and the second catalytic species providing epoxidation activity and comprising silver. Preferably, both the dehydrogenation and epoxidation catalytic species are supported on a single support. Other preferred features of the catalyst system, such as preferred metals for the dehydrogenation catalyst, are as described for the first aspect of the present invention.

### Example

### Catalyst Preparation

### 1) Preparation of Ni-Nb/γ-alumina

A catalyst of mixed Ni-Nb-O oxides supported on γ-alumina (theoretical metal loading: %Ni = 15 and %Nb = 4,02) with an Nb/Ni atomic ratio equal to 0.176 was prepared by the evaporation method. Aqueous solutions containing the precursor salts, 1.666 g of nickel nitrate hexahydrate (>99%, Merck) and 0.323 g ammonium niobium oxalate (99.99%, Aldrich), in appropriate amounts were heated at 70 °C under continuous stirring for 1 h to ensure complete dissolution and good mixing of the starting compounds. Catalysts were prepared by conventional wet impregnation of the γ-Al₂O₃ support 2,33 g (Degussa, 100 m²/g) with aqueous solutions of nickel nitrate, nickel nitrate and ammonium niobium oxalate at 25 °C under continuous stirring for 30 min

The solvent was then removed by evaporation under reduced pressure, and the resulting solids were dried overnight at 120 °C and calcined in synthetic air at 450 °C for 5 h and oxygen at 450 °C for 30 min.

### 2) Preparation of Ag/γ-alumina

The catalysts were prepared by conventional wet impregnation of 5 g of the γ-Al₂O₃ support (Degussa, 100 m²/g) with aqueous solutions of silver nitrate, 1.181g of AgNO₃ 99.995% (Aldrich) (theoretical metal loading %Ag=15). After impregnation, the solvent was removed by evaporation under reduced pressure, and the resulting solid was dried overnight at 120 °C and calcined in synthetic air at 450 °C for 5 h.

### Catalyst screening

The catalytic performance was measured by intimately mixing the Ni-Nb and Ag catalysts in a 1:1 ratio in a batch reactor. A reaction mixture comprising 50% C₂H₆, 10% O₂ and 40% N₂ (by volume) was passed over the catalyst mixture at 350°C and 10 bar pressure. After 30 minutes of reaction the products were analysed and ethylene oxide was observed.

Comparative examples were performed under identical conditions using single beds of the respective Ni-Nb and Ag catalysts. No ethylene oxide was observed over the Ni-Nb catalyst. Small amounts of ethylene oxide were observed from the Ag catalyst alone, but significantly less than from the mixed catalyst

## Claims

1. A process for the production of an epoxide from a mixture comprising an alkane and an alkene, which process comprises contacting said mixture comprising said alkane and said alkene and a source of oxygen with a catalyst system comprising a mixture of at least two catalytic species, the first catalytic species providing dehydrogenation activity and the second catalytic species providing epoxidation activity and comprising silver.

2. A process according to claim 1 wherein a product stream comprising unreacted alkane, alkene and epoxide is formed, and the unreacted alkane and alkene are separated from the product stream and recycled.

3. A process according to claim 1 or claim 2 wherein the mixed catalyst bed comprises an intimate mixture of two catalysts on separate particles of suitable supports.

4. A process according to claim 1 or claim 2 wherein the mixed catalyst bed comprises both dehydrogenation and epoxidation catalysts supported at different locations on a single support.

5. A process according to any of the preceding claims wherein the dehydrogenation catalyst is supported or unsupported metal oxide catalyst.

6. A catalyst system which is a mixture of at least two catalytic species, the first catalytic species providing dehydrogenation activity and the second catalytic species providing epoxidation activity and comprising silver.

7. A catalyst system as claimed in claim 6 wherein both the dehydrogenation and epoxidation catalytic species are supported on a single support.
